# EUROPÄISCHE PATENTSCHRIFT

(11) **EP 2 162 530 B1**
(45) Veröffentlichungstag und Bekanntmachung des Hinweises auf die Patenterteilung: **07.06.2017**
(21) Anmeldenummer: 08707810.1
(22) Anmeldetag: 27.02.2008
(51) Int. Cl.: C12N 5/071

(54) **ISOLIERTE PROLIFERIERENDE ZELLEN MIT STAMMZELLEIGENSCHAFTEN AUS ADULTEM GEWEBE VON WECHSELWARMEN WIRBELTIEREN, STABILE ZELLKULTUREN DAVON UND VERFAHREN ZU DEREN HERSTELLUNG**
ISOLATED PROLIFERATING CELLS WITH STEM CELL PROPERTIES FROM ADULT TISSUE OF POIKILOTHERMIC VERTEBRATES, STABLE CELL CULTURES THEREOF, AND METHOD FOR THEIR PREPARATION
CELLULES ISOLÉES PROLIFÉRANTES DOTÉES DE PROPRIÉTÉS DE CELLULES SOUCHES DE TISSUS ADULTES DE VERTÉBRÉS À SANG FROID, CULTURES STABLES DE CES CELLULES ET PROCÉDÉ POUR LEUR PRÉPARATION

(30) Priorität: 27.06.2007 DE 102007029699
(43) Veröffentlichungstag der Anmeldung: 17.03.2010
(73) Patentinhaber: Fraunhofer-Gesellschaft zur Förderung der angewandten Forschung e.V., 80686 München (DE)
(72) Erfinder: KRUSE, Charli, 23923 Herrnburg (DE); GÜRLEYIK, Emel, 23558 Lübeck (DE)
(74) Vertreter: Katzameyer, Michael
(86) Internationale Anmeldenummer: PCT/EP2008/001550
(87) Internationale Veröffentlichungsnummer: WO 2009/000347

(56) Entgegenhaltungen:
- KOBAYASHI ISAO ET AL: "Demonstration of hematopoietic stem cells in ginbuna carp (Carassius auratus langsdorfii) kidney." DEVELOPMENTAL AND COMPARATIVE IMMUNOLOGY 2006, Bd. 30, Nr. 11, 2006, Seiten 1034-1046, XP002485082 ISSN: 0145-305X
- MORITOMO TADAAKI ET AL: "Cell culture of clonal ginbuna crucian carp hematopoietic cells: differentiation of cultured cells into erythrocytes in vivo" DEVELOPMENTAL & COMPARATIVE IMMUNOLOGY, Bd. 28, Nr. 9, 1. Juli 2004 (2004-07-01), Seiten 863-869, XP002485083 ISSN: 0145-305X
- DAVIDSON ALAN J ET AL: "The 'definitive' (and 'primitive') guide to zebrafish hematopoiesis" ONCOGENE, Bd. 23, Nr. 43, 20. September 2004 (2004-09-20), Seiten 7233-7246, XP002485084 ISSN: 0950-9232
- ANDERSON M J ET AL: "Molecular differentiation of neurons from ependyma-derived cells in tissue cultures of regenerating teleost spinal cord", MOLECULAR BRAIN RESEARCH, ELSEVIER SCIENCE BV, AMSTERDAM, NL, vol. 2, no. 2, 1 July 1987 (1987-07-01), pages 131-136, XP025435654, ISSN: 0169-328X, DOI: 10.1016/0169-328X(87)90006-4 [retrieved on 1987-07-01]

## Beschreibung

Die Erfindung betrifft ein Verfahren zur Herstellung von isolierten proliferierenden Zellen mit Stammzelleigenschaften und der entsprechenden stabilen Zellkulturen aus adultem Gewebe von Fischen (*Pisces*) sowie die damit hergestellten Zellen und Zellkulturen.

Als Stammzellen werden solche Zellen bezeichnet, die die Fähigkeit besitzen, sich unbegrenzt zu teilen und sich unter geeigneten Umständen bzw. durch geeignete Stimuli in verschiedene Zellarten differenzieren zu können. Stammzellen besitzen das Potenzial, sich in Zellen mit charakteristischer Gestalt und spezialisierten Funktionen zu entwickeln. Diese spezialisierten Zellen sind häufig nur sehr begrenzt oder gar nicht außerhalb des Organismus zu kultivieren, so dass sie über den Umweg der Kultur von Stammzellen hergestellt werden müssen.

Embryonale Stammzellen konnten bisher von einigen wenigen Fischarten (Zebrafisch und Medaka) isoliert und untersucht werden [Ma et al., 2001, Proc. Natl. Acad. Sci. U. S. A. 98:2461-2466; Epub 2001, 13. Feb.; Hong et al., 1996, Mech. Develop., 60:33-44], während über adulte Stammzellen aus Fischen nur sehr wenige Untersuchungen bekannt sind [Tawk und Vriz 2003, Med. Sci. (Paris) 19:465-471; Alvarado und Tsonis al., 2006, Nat. Rev. Genet., 7:873-884]. Diese Untersuchungen wurden jedoch an komplexen Fischgeweben, in der Regel *in vivo* an Flosse, Herz und Hirn, vorgenommen und ermöglichten nicht die Gewinnung isolierter Stammzellen oder stabiler Stammzellkulturen.

In Developmental and Comparative Immunologie, Bd. 31, Nr. 7, 696-707, (2007) wird die Isolierung und Kultivierung von hematopoetischen Stammzellen aus dem Nierengewebe von Karpfen beschrieben. Die erhaltenen Stammzellen haben nicht das Vermögen zur Differenzierung in Zellen verschiedener Keimblätter. Außerdem wird die Kopfniere aufgrund des äußerst geringen Vorkommens (nahe 0 %) als ungeeignetes Ausgangsmaterial zur Isolation der betreffenden Stammzellen genannt (Abstract und Seite 703).

Der Erfindung liegt daher die Aufgabe zu Grunde, isolierte proliferierende Zellen mit Stammzelleigenschaften, insbesondere unbeschränkte Teilungsfähigkeit und Multipotenz oder Pluripotenz, aus adultem Gewebe von Fischen zu gewinnen und stabile proliferierende Zellkulturen davon etablieren, mit denen ein breites Spektrum verschiedener differenzierter Zellen produziert werden kann.

Die Aufgabe wird erfindungsgemäß gelöst durch die Bereitstellung des Verfahrens nach Anspruch 1 sowie der Zellen nach Anspruch 4 und Zellkultur nach Anspruch 7. Weitere Aspekte und/oder bevorzugte Ausführungsformen der Erfindung sind Gegenstand der weiteren Ansprüche.

Der Fisch ist insbesondere ein Nutzfisch. Einige nichtbeschränkende Beispiele für geeignete Spezies sind Stör, Hering, Forelle, Lachs, Aal, Karpfen.

Als adultes Ausgangsgewebe wird Kopfnierengewebe verwendet. Die Kopfniere hat in Fischen blutbildende Funktionen. Von den blutbildenden Stammzellen der Säuger ist bekannt, dass sich diese nach Differenzierung in reife Blutzellen unter Zellkulturbedingungen vom Untergrund lösen. Im Gegensatz dazu lösten sich die erfindungsgemäß präparierten Zellen aus Fischkopfnieren nicht, hafteten auch nach mehr 18 Passagen noch am Untergrund und wiesen Merkmale von Stammzellen auf, ähnlich denen, die aus exokrinen Drüsen isoliert wurden. Dieses Ergebnis war nicht zu erwarten.

Die erfindungsgemäß bereitgestellten proliferierenden Zellen haben Stammzelleigenschaften, d.h., sie besitzen eine uneingeschränkte Teilungsfähigkeit, und sind darüber hinaus multipotent oder pluripotent, d.h., sie können spontan in Zellen von mindestens zwei Keimblättern (Ektoderm und Mesoderm) differenzieren.

Es ist ausgesprochen überraschend, dass das erfindungsgemäße einfache Verfahren die Herstellung adulter Stammzellen ermöglicht, die in ihrem Differenzierungspotenzial verschiedene Keimblätter widerspiegeln und daher wenigstens als multipotent bezeichnet werden können. Die mit dem erfindungsgemäßen Verfahren erzeugten Stammzellen sind leicht zu gewinnen und differenzieren reproduzierbar spontan in verschiedene Zelltypen. Bisher nachgewiesen wurden Muskelzellen, Gliazellen, Epithel- und Endothelzellen, die erhältlichen Zellarten sind jedoch sicherlich nicht darauf beschränkt und es liegen bereits Befunde vor, die auf weitere Zellarten hindeuten.

Weder zur Kultivierung der Stammzellen noch zur Differenzierung ist eine Feederzellschicht (*Feeder Layer*) notwendig und die Zellen müssen auch nicht transplantiert werden, um zu differenzieren. Der Begriff Feederzellen, wie in der vorliegenden Anmeldung verwendet, umfasst alle Zellen, die das Wachstum der eigentlich zu kultivierenden Zellen dadurch fördern, dass sie Wachstumsfaktoren freisetzen und/oder eine extrazelluläre Matrix bereitstellen, bzw. die Differenzierung der Stammzellkultur verhindern.

Bemerkenswert ist auch, dass die erfindungsgemäß hergestellten Stammzellen in Adhäsionskultur proliferieren können. Im Gegensatz dazu werden herkömmliche hematopoetische Stammzellen in der Regel in Suspensionskultur gezüchtet.

In Stammzellkulturen der Erfinder haben die Zellen nach bisher mehr als 25 Passagen über 6 Monate lang ihre Fähigkeit zur Selbsterneuerung und uneingeschränkten Teilung behalten und die Kulturen sind weiterhin stabil.

Darüber hinaus können die erfindungsgemäß erhaltenen Stammzellen eingefroren (vorzugsweise in flüssigem Stickstoff) gelagert werden und behalten ihre Differenzierbarkeit und Vitalität unverändert bei.

Die erfindungsgemäßen Zellen und Zellkulturen können bereits bei tieferen Temperaturen als 37°C (typisch für Säugerzellkulturen) kultiviert werden. Der geeignete Temperaturbereich hängt von der Art und Herkunft der als Gewebequelle verwendeten Fischspezies ab und kann daher ein breites Spektrum von etwa 1-35°C umfassen. Bei tropischen Fischen als Ausgangsorganismus kann eine geeignete Kultivierungstemperatur im oberen Bereich liegen, z.B. etwa 25-35°C betragen, während sie für Zellen von Fischen aus kalten Regionen z.B. 1-10°C betragen kann. Insbesondere für Zellen von Fischen aus mittleren Breiten wird der Temperaturbereich typischerweise in einem Bereich von 5-27 °C, vorzugsweise 10-25°C, bevorzugter 17-24°C, insbesondere 18-20°C oder etwa Raumtemperatur, liegen. Den jeweils geeigneten oder optimalen Temperaturbereich kann der Fachmann unschwer durch Routineversuche feststellen.

Die erfindungsgemäß hergestellten Stammzellen und Stammzellkulturen haben ein breites Spektrum von Anwendungen.

Ein Beispiel hierfür ist die Bereitstellung komplexer zellulärer Testsysteme, z.B. zur Auswertung zellulärer Interaktionen oder zur Analyse der Wirkung verschiedener Noxen, Chemikalien, insbesondere potentieller Arzneiwirkstoffe, oder auch Kosmetika.

Vorzugsweise umfassen diese zellulären Systeme mehrere Arten von Zellen, z.B. solche die auch in natürlichen Geweben nebeneinander vorkommen, die damit auch gleichzeitig getestet werden können.

Die erfindungsgemäß hergestellten Stammzellen und Stammzellkulturen können auch zur Entwicklung gewebeähnlicher oder organähnlicher multizellulärer Systeme, vorzugsweise aus mehreren Zellarten, *in vitro* eingesetzt werden.

Ferner können diese für die Nahrungs- und Futterproduktion verwendet werden. Sie können beispielsweise dazu verwendet werden, fischspezifische Biomasse herzustellen, wie z.B. künstliches Fleisch, Fischmehl, Fischöl oder einzelne Bestandteile dieser Zellen, wie z.B. Omega-Fettsäuren oder Proteine mit einer für Fische typischen Aminosäurezusammensetzung.

Eine allgemeinere Anwendung für diese Zellen, Zellkulturen und multizellulären Systeme ist die Verwendung als *in vitro-*System zur Produktion gewünschter Substanzen *in vitro*.

Bei diesen Substanzen kann es sich um bestimmte Substanzen handeln, die natürlicherweise in den Ausgangsorganismen, z.B. Fischen, produziert werden. Ein spezielles Beispiel hierfür sind besondere Proteine, z.B. kälteadaptierte Enzyme mit einem Aktivitätsoptimum bei niedrigen Temperaturen (z.B. etwa 10°C), u.a. mit Einsatzmöglichkeiten in der Nahrungsmittelreifung. Solche Enzyme könnten z.B. von Drüsenzellen produziert werden, die durch spontane oder gezielte Differenzierung aus den erfindungsgemäßen Stammzellen gebildet werden.

Die erfindungsgemäß hergestellten Stammzellen und Zellkulturen können auch als Empfängerzellen oder Vehikel zur Aufnahme von fremden Genmaterial oder anderem Fremdmaterial nach im Stand der Technik grundsätzlich bekannten Verfahren verwendet werden. Die Expression des fremden Genmaterials erlaubt dann die Produktion heterogener Proteine in diesen Zellen und Kulturen.

Ein besonderer Vorteil solcher Zellen und Zellkulturen besteht darin, dass sie bereits bei tieferen Temperaturen als 37°C (typisch für Säugerzellkulturen), z.B. in einem Bereich von 1-35°C, vorzugsweise 5-27°C oder 17-24°C, insbesondere 18-20°C oder etwa Raumtemperatur, gut wachsen, was eine energieintensive Heizung unnötig macht.

Eine noch weitere Anwendungsmöglichkeit der erfindungsgemäßen Zellen ist das reproduktive Klonen von wechselwarmen Wirbeltieren, insbesondere zur Fischzucht (Klonfische).

Eine Ausführungsform der vorliegenden Erfindung betrifft auch einen Kit, umfassend die Zellen nach einem der Ansprüche 4 bis 6 sowie weitere Hilfsmittel oder Reagenzien, z.B. Reagenzien zur Kultivierung der Zellen oder diagnostische Reagenzien.

### FIGURENBESCHREIBUNG

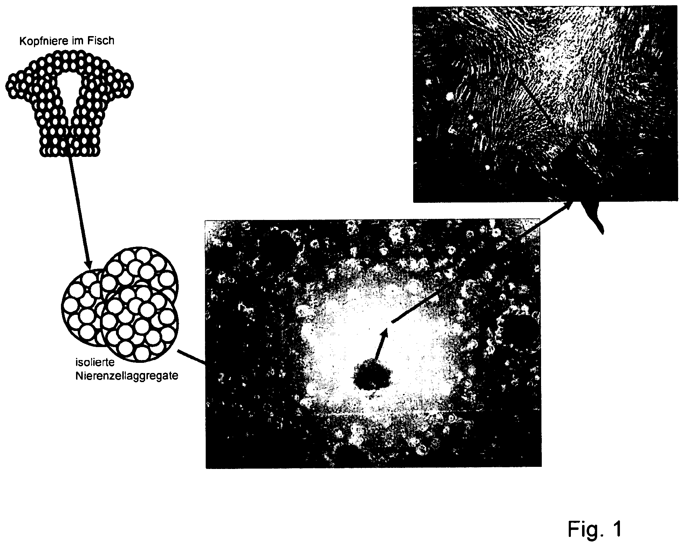

Fig. 1 zeigt eine schematische Darstellung des Ablaufs des Verfahrens ausgehend von der Kopfniere eines Fisches.

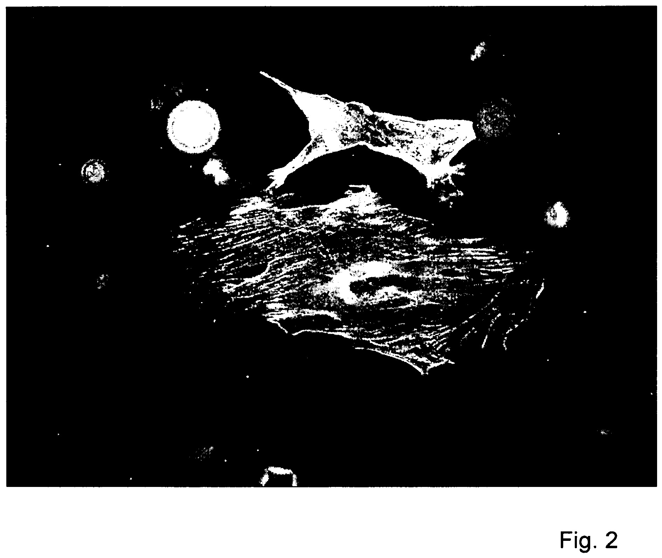

Fig. 2 zeigt den immunhistologischen Nachweis von nach dem erfindungsgemäßen Verfahren hergestellten differenzierten Zellen (Muskelzellen) mit mesodermalen Eigenschaften aus Stören. Die Zellen wurden mit Antikörpern gegen glattes Muskelaktin (α-smooth-muscle-actin, SMA) angefärbt. Zusätzlich wurden die Zellkerne mit DAPI angefärbt.

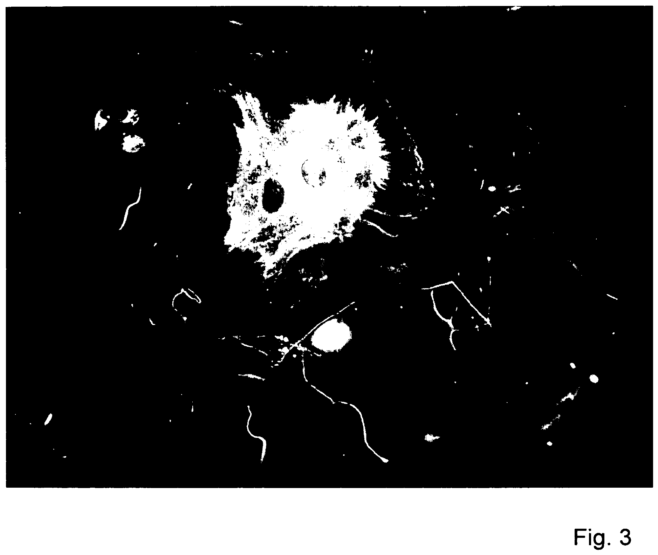

Fig. 3 zeigt den immunhistologischen Nachweis von nach dem erfindungsgemäßen Verfahren hergestellten Gliazellen mit ektodermalen Eigenschaften aus Stören. Die Zellen wurden mit Antikörpern gegen gliales fibrilläres saures Protein (glial fibrillary acidic protein, GFAP) angefärbt. Zusätzlich wurden die Zellkerne mit DAPI angefärbt.

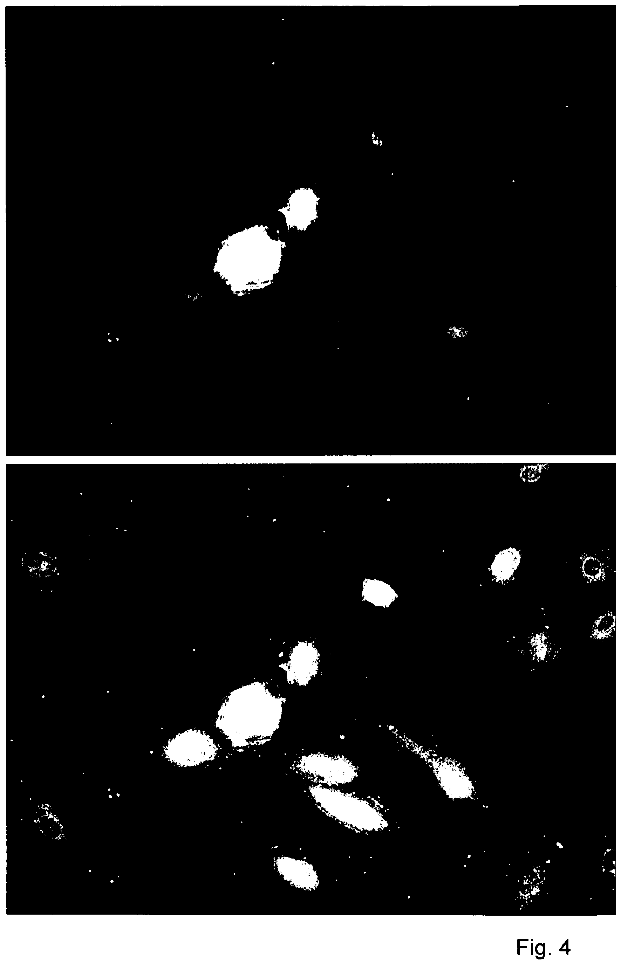

Fig. 4 zeigt den immunhistologischen Nachweis von nach dem erfindungsgemäßen Verfahren hergestellten differenzierten Zellen aus Stören mit Strukturproteinen (Cytokeratin) und dem Translationsmarker Vigilin. Die Zellen wurden mit Antikörpern gegen Pancytokeratin (rot) und Vigilin (grün) angefärbt. Zusätzlich wurden die Zellkerne mit DAPI angefärbt.

Die Cytokeratin-Strukturproteine werden vor allem in epithelialen und endothelialen Zellen gebildet. Das Protein Vigilin ist typisch für proteinsynthetisierende Zellen und wurde bisher in allen Stammzellen gefunden.

Neben diesen Nachweisen konnte auch gezeigt werden, dass Zellen vorhanden sind, die Fetttröpfchen einlagern und ein Vielzahl unterschiedlicher Morphologien der Zellen zu erkennen sind, die auf eine breitere Vielfalt von Zellen hinweisen (Daten nicht gezeigt).

Entsprechend dem in Fig. 1 gezeigten Schema wird zur Gewinnung der Zellen Kopfnierengewebe mechanisch und enzymatisch zerkleinert in Kultur genommen. Dabei werden keine kompletten Gewebeblöcke in Kultur genommen, sondern intakte Zellaggregate, die kleiner als ca. 200 µm sind, in Kulturgefäße gegeben, aus denen dann die gesuchten Zellen auswachsen oder sich neu bilden.

Über mehrere Wochen werden diese Zellen und Zellverbände in Kulturgefäßen kultiviert. Alle 2-3 Tage wird das Medium gewechselt, wobei alle differenzierten Zellen entfernt werden. Bei den in Kultur persistierenden Zellen handelt es sich um undifferenzierte Zellen mit uneingeschränkter Teilungsfähigkeit.

Hervorzuheben ist hier, dass zum einen der enzymatische Gewebeverdau bei 37°C stattfindet, was die Zellen anscheinend vertragen, und dass zum anderen die Zellen sehr gut bei 18°-20°C wachsen, während die Ausgangstiere selbst oftmals bei niedrigeren Temperaturen optimal gedeihen.

Derartige primäre Zellen mit einer sehr hohen Vermehrungsfähigkeit aus Kopfnieren von Fischen sind noch nicht beschrieben worden. Bisher demonstrierten diese Zellen eine uneingeschränkte Teilungsfähigkeit und konnten ca. 6 Monate ununterbrochen in Kultur gehalten werden.

In dem folgenden nicht beschränkenden Beispiel sollen die vorzugsweise verwendeten Arbeitsschritte des Verfahrens näher dargelegt werden.

Die allgemeinen Arbeitsanweisungen, wie sie für Verfahren zur Kultivierung von tierischen Zellen und insbesondere von Säugetierzellen gebräuchlich sind, sind zu beachten. Eine sterile Umgebung, in der das Verfahren durchgeführt werden soll, ist - auch wenn hierzu keine weitere Beschreibung erfolgt - in jedem Fall einzuhalten. Folgende Puffer und Medien müssen bereitgestellt werden:

| | |
|---|---|
| HEPES-Stammlösung (pH 7,6) | 2,383 g HEPES auf 100 ml *A*. *bidest.* |
| HEPES-Eagle-Medium | 90 ml Modified Eagle Medium (MEM) |
| | 10 ml HEPES-Stammlösung |
| Isolationsmedium (pH 7,4) | 32 ml HEPES-Eagle-Medium |
| | 8 ml 5% BSA in A. bidest. |
| | 200 µl 0,1 M CaCl₂ |
| | 100 µl Trasylol (200.000 KIE) |
| Digestionsmedium (pH 7,4) | 20 ml Isolationsmedium |
| | 4 mg Kollagenase |
| Inkubationsmedium | Dulbecco's Modified Eagle Medium (DMEM) |
| Differenzierungsmedium | 380 ml DMEM |
| | 100 ml 30 min bei 54 °C inaktiviertes FCS (oder Pilz- bzw. Algenextrakte) |
| | 5 ml Glutamin (PAA Laboratories GmbH) |
| | 5 ml (3,5 µl ß-Mercaptoethanol auf 5 ml PBS) |
| | 5 ml Non-essential amino acids (GIBCO BRL) |
| | 5 ml Penicillin/Streptomycin (PAA) |

### BEISPIEL 1

### 1. Präparation des Gewebes und Isolierung der Zellen

Die Kopfniere eines Störs wird von der Oberfläche der oberen Körperhöhle gleich hinter dem Schädel beginnend entfernt. Danach wird das Gewebe in einem Becherglas mit Digestionsmedium mit einer feinen Schere zerkleinert. Die Suspension wird anschließend 1 min mit Carbogen begast und für 20 min bei 37 °C in einem Schüttler bei 200 Zyklen/min inkubiert. Danach saugt man das Medium vorsichtig ab und wäscht die Gewebestücke zweimal mit je 10 ml Isolationsmedium und gibt wieder 5-10 ml Digestionsmedium zum Gewebe hinzu.

Nach erneutem Begasen mit Carbogen für 1 min und Inkubation für 15 min bei 37 °C in einem Schüttler bei 200 Zyklen/min werden die Gewebestücke durch sukzessives Aufziehen in jeweils einer 10 ml, 5ml und 2 ml Glaspipette zerkleinert und durch einlagiges Filtergewebe mit einer Maschenweite von ca. 200µm gepresst. Die so vereinzelten Zellen werden nun 1-2 mal in Inkubationsmediüm gewaschen (37 °C) und jedes Mal 5 min bei 90 g (800 UPM) zentrifugiert. Das zuletzt erhaltene Pellet wird in Inkubationsmedium resuspendiert und auf Gewebekulturschalen (25 cm²) verteilt.

### 2. Kultivierung der Zellen

Die Gewebekulturschalen mit den isolierten Zellen werden im Brutschrank bei 18-20°C und 5 % CO₂ kultiviert. Alle 3-4 Tage wird das Medium gewechselt. Dabei werden alle nichtadhärenten und viele differenzierten Zellen entfernt.

Nachdem die Zellen den Boden komplett bedecken (also konfluent sind), erstmals etwa am 10. Tag der Kultur, werden die Zellen mit einer Lösung bestehend aus 2 ml PBS, 1 ml Trypsin und 2 ml Inkubationsmedium bei 37°C passagiert. Dabei lösen sich die Zellen vom Boden der Kulturschale. Die Zellsuspension wird 5 Minuten zentrifugiert, der Überstand abgesaugt und die Zellen in 5 ml Inkubationsmedium resuspendiert, auf eine mittlere Zellkulturflasche (75 cm²) überführt und 10 ml Inkubationsmedium dazugegeben. Der Medienwechsel erfolgt alle drei Tage.

Am vierzehnten Tag in Kultur werden die Zellen erneut, aber diesmal mit 6 ml PBS, 2 ml Trypsin und 6 ml Inkubationsmedium, passagiert. Die Zellsuspension wird 5 Minuten zentrifugiert, der Überstand abgesaugt und die Zellen in 15 ml Inkubationsmedium resuspendiert, auf 3 mittlere Zellkulturflaschen überführt und jeweils 10 ml Inkubationsmedium dazugegeben.

Die Zellen werden weiter kultiviert und so oft passagiert und ausgesät bis die Zellen einen semikonfluenten bis konfluenten Zustand erreichen.

Zur Differenzierung können die Zellen in Differenzierungsmedium, (z.B. mit der in der obigen Tabelle angegebenen Zusammensetzung) überführt und unter bekannten Kultivierungsbedingungen differenziert werden. Für die Differenzierung sind zusätzliche Differenzierungs- und Wachstumsfaktoren nicht unbedingt erforderlich, da die erfindungsgemäßen Zellen spontan in verschiedene Zelltypen differenzieren können. Trotzdem kann es zur Förderung einer gezielten Differenzierung in bestimmte Zelltypen vorteilhaft sein, solche Differenzierungs- und Wachstumsfaktoren zuzugeben. Derartige Faktoren, die spezifisch die Differenzierung von Stammzellen in bestimmte Zelltypen fördern, sind im Stand der Technik für eine ganze Reihe von Zelltypen bekannt und können analog zu bekannten Protokollen eingesetzt werden.

## Patentansprüche

1. Verfahren zur Herstellung adulter proliferierender Zellen mit Stammzelleigenschaften, welche mindestens in Zellen der beiden Keimblätter Ektoderm und Mesoderm differenzieren können, umfassend
Entnehmen von Gewebe aus der Kopfniere eines Fisches, Zerkleinern des entnommenen Gewebes,
Kultivieren des zerkleinerten Gewebes, und
Vermehren der in Kultur persistierenden Zellen,
wobei das Gewebe so schonend zerkleinert wird, dass Zellverbände, die kleiner als etwa 200 µm sind, in den resultierenden Gewebestückchen weitgehend erhalten bleiben,
und das zerkleinerte Gewebe in adhärenter Kultur kultiviert und die in Kultur persistierenden adhärenten Zellen weiter kultiviert und nicht-adhärente differenzierte Zellen durch Mediumwechsel abgetrennt werden.

2. Verfahren nach Anspruch 1, **dadurch gekennzeichnet, dass** das zerkleinerte Gewebe zunächst unter geeigneten Bedingungen in Gewebekulturgefäßen kultiviert wird, wobei der Großteil der differenzierten Zellen schnell im Verlauf weniger Tage abstirbt und sich von den Stammzellen löst, wonach die Stammzellen sich am Boden des Gewebekulturgefäßes festsetzen,
und das restliche Gewebe und nicht-adhärente differenzierte Zellen durch einen ersten Mediumwechsel weitgehend abgetrennt werden, und
durch weitere Medienwechsel in Abständen von wenigen Tagen, vorzugsweise etwa 2-3 Tagen, restliche nicht-adhärente Zellen abgetrennt werden.

3. Verfahren nach einem der Ansprüche 1 bis 2, **dadurch gekennzeichnet, dass** die Kultivierung und Vermehrung der in Kultur persistierenden Zellen bei 5-27 °C, insbesondere 17-24 °C, erfolgt.

4. Proliferierende Zellen mit Stammzelleigenschaften, erhältlich mit dem Verfahren nach einem der Ansprüche 1-3 aus dem Kopfnierengewebe eines Fisches, **dadurch gekennzeichnet, dass** die Zellen uneingeschränkte Teilungsfähigkeit aufweisen und mindestens in Zellen der beiden Keimblätter Ektoderm und Mesoderm differenzieren können.

5. Zellen nach Anspruch 4, **dadurch gekennzeichnet, dass** die Zellen auch nach dem Einfrieren/Kryokonservieren ihre Fähigkeit zur Selbsterneuerung und uneingeschränkten Teilung behalten und nicht differenzieren.

6. Zellen nach Anspruch 4 oder 5, **dadurch gekennzeichnet, dass** die Zellen das Vermögen zur Proliferation in Adhäsionskultur aufweisen.

7. Zellkultur, insbesondere Adhäsionskultur, umfassend die Zellen nach einem der Ansprüche 4 bis 6 in einem Kulturmedium, welches die stabile Aufrechterhaltung und Vermehrung der Zellen im wesentlichen ohne Differenzierung erlaubt.

8. Zellkultur nach Anspruch 7, **dadurch gekennzeichnet, dass** die Zellen für mehr als 25 Passagen ihre Fähigkeit zur Selbsterneuerung und uneingeschränkten Teilung behalten.

9. Differenzierungskultur, umfassend die Zellen nach einem der Ansprüche 4 bis 6 sowie daraus differenzierte Zellen in einem Differenzierungsmedium, **dadurch gekennzeichnet, dass** das Differenzierungsmedium keine zusätzlichen Wachstums- oder Differenzierungsfaktoren umfasst.

10. Verwendung der Zellen nach einem der Ansprüche 4 bis 6 oder der Zellkultur nach einem der Ansprüche 7 bis 8 zur Entwicklung gewebeähnlicher oder organähnlicher multizellulärer Systeme *in vitro*.

11. Verwendung der Zellen nach einem der Ansprüche 4 bis 6 oder der Zellkultur nach einem der Ansprüche 7 bis 8 als *in vitro*-System zum Testen von Chemikalien, insbesondere zum Screenen von Arzneiwirkstoffen, oder als *in vitro-*System zur Produktion gewünschter Substanzen *in vitro*.

12. Verwendung nach Anspruch 11, **dadurch gekennzeichnet, dass** Proteine, insbesondere Enzyme mit einem Aktivitätsoptimum bei niedrigen Temperaturen, Fischöle oder Fettsäuren produziert werden.

13. Verwendung der Zellen nach einem der Ansprüche 4 bis 6 oder der Zellkultur nach einem der Ansprüche 7 bis 8 für das reproduktive Klonen von wechselwarmen Wirbeltieren, insbesondere zur Fischzucht, oder als Empfängerzellen oder Vehikel zur Aufnahme von fremdem Genmaterial oder anderem Fremdmaterial.

14. Kit, umfassend die Zellen nach einem der Ansprüche 4 bis 6 sowie weitere Hilfsmittel oder Reagenzien, z.B. Reagenzien zur Kultivierung der Zellen oder diagnostische Reagenzien.

## Claims

1. A method for the preparation of adult proliferating cells with stem cell properties, which can differentiate into cells of at least both of the ectodermal and mesodermal germ layers, comprising
removing tissue from the pronephros of a fish, comminuting the removed tissue,
cultivating the comminuted tissue, and
propagating the cells persisting in culture,
wherein the tissue is gently comminuted such that cell aggregates smaller than about 200 µm are largely maintained in the resulting small tissue pieces,
and the comminuted tissue is cultivated in adhesion culture and the adherent cells persisting in the culture are further cultivated and non-adhering differentiated cells are separated by a replacement of medium.

2. The method according to claim 1, **characterized in that** the comminuted tissue is initially cultivated under suitable conditions in tissue culture vessels, the majority of the differentiated cells dying quickly over the course of a few days and detaching from the stem cells, the stem cells subsequently adhering on the bottom of the tissue culture vessel,
and the remaining tissue and non-adhering differentiated cells are largely separated by means of a first medium replacement, and
remaining non-adhering cells are being separated by means of further medium replacements within the space of a few days, preferably about 2-3 days.

3. The method according to one of claims 1 to 2, **characterized in that** the cultivating and propagating of the cells persisting in culture take place at 5-27°C, in particular 17-24°C.

4. Proliferating cells with stem cell properties, obtainable by the method according to one of claims 1-3 from the pronephros tissue of a fish, **characterized in that** the cells feature unlimited divisibility and can differentiate into cells of at least both of the ectodermal and mesodermal germ layers.

5. The cells according to claim 4, **characterized in that** the cells maintain their capability to self-renew and divide unlimitedly even after freezing/cryoconservation and do not differentiate.

6. The cells according to claim 4 or 5, **characterized in that** the cells have the capability to proliferate in an adhesion culture.

7. A cell culture, in particular an adhesion culture, comprising the cells according to one of claims 4 to 6 in a culture medium which permits the stable maintenance and propagation of the cells, substantially without differentiation.

8. The cell culture according to claim 7, **characterized in that** the cells retain their capability to self-renew and divide unlimitedly for more than 25 passages.

9. A differentiation culture comprising the cells according to one of claims 4 to 6 and cells differentiated therefrom in a differentiation medium, **characterized in that** the differentiation medium comprises no additional growth or differentiation factors.

10. Use of the cells according to one of claims 4 to 6 or of the cell culture according to one of claims 7 to 8 for the development of tissue-like or organ-like multicellular systems *in vitro*.

11. Use of the cells according to one of claims 4 to 6 or of the cell culture according to one of claims 7 to 8 as an *in vitro* system for the testing of chemicals, in particular for the screening of pharmaceutical drugs, or as an *in vitro* system for the production of desired substances *in vitro*.

12. The use according to claim 11, **characterized in that** proteins, in particular enzymes with an activity optimum at low temperatures, fish oils or fatty acids are produced.

13. Use of the cells according to one of claims 4 to 6 or of the cell culture according to one of claims 7 to 8 for the reproductive cloning of poikilothermic vertebrates, in particular for fish breeding, or as recipient cells or vehicles to receive foreign gene material or other foreign matter.

14. A kit comprising the cells according to one of claims 4 to 6, and further expedients or reagents, e.g. reagents for the cultivation of the cells or diagnostic reagents.

## Revendications

1. Procédé servant à fabriquer des cellules adultes proliférantes présentant des propriétés de cellules souches, qui peuvent être différenciées au moins en cellules des deux foyers embryonnaires ectoderme et mésoderme, comprenant les étapes consistant à :
prélever du tissu du rein antérieur d'un poisson,
fractionner le tissu prélevé,
cultiver le tissu fragmenté, et
multiplier les cellules persistantes en culture,
dans lequel le tissu est fragmenté avec ménagement de sorte que des amas cellulaires qui sont inférieurs à environ 200 µm sont largement préservés dans les fragments de tissus résultant,
et le tissu fractionné est cultivé en une culture adhérente et les cellules adhérentes persistantes en culture continuent à être cultivées et les cellules différenciées non adhérentes sont séparées par un changement de milieu.

2. Procédé selon la revendication 1, **caractérisé en ce que** le tissu fractionné est cultivé dans un premier temps dans des conditions adaptées dans des récipients de culture de tissu, dans lequel la majeure partie des cellules différenciées meurt rapidement en quelques jours et se détache des cellules souches, les cellules souches se fixant après quoi au niveau du fond du récipient de culture de tissu,
et le tissu restant et des cellules différenciées non-adhérentes sont largement séparés par un premier changement de milieu, et
des cellules non-adhérentes restantes sont séparées par d'autres changements de milieux à des intervalles de quelques jours, de préférence d'environ 2 - 3 jours.

3. Procédé selon l'une quelconque des revendications 1 à 2, **caractérisé en ce que** la mise en culture et la multiplication des cellules persistantes en culture sont effectuées à une température de 5-27 °C, en particulier de 17-24 °C.

4. Cellules proliférantes présentant des propriétés de cellules souches, pouvant être obtenues à l'aide du procédé selon l'une quelconque des revendications 1-3 à partir du tissu de rein antérieur d'un poisson, **caractérisées en ce que** les cellules présentent une capacité de division illimitée et peuvent être différenciées au moins en des cellules des deux foyers embryonnaires, ectoderme et mésoderme.

5. Cellules selon la revendication 4, **caractérisées en ce que** les cellules conservent également après la congélation/cryoconservation leur capacité d'autorenouvellement et de division illimitée et ne sont pas différenciées.

6. Cellules selon la revendication 4 ou 5, **caractérisées en ce que** les cellules présentent l'aptitude à la prolifération en culture d'adhésion.

7. Culture de cellules, en particulier culture d'adhésion, comprenant les cellules selon l'une quelconque des revendications 4 à 6 dans un milieu de culture, qui permet la conservation stable et la multiplication des cellules sensiblement sans différenciation.

8. Culture de cellules selon la revendication 7, **caractérisée en ce que** les cellules conservent leur capacité à l'autorenouvellement et à la division illimitée pour plus de 25 passages.

9. Culture de différenciation, comprenant les cellules selon l'une quelconque des revendications 4 à 6 ainsi que des cellules différenciées en résultant dans un milieu de différenciation, **caractérisée en ce que** le milieu de différenciation ne comprend aucun facteur de croissance ou de différenciation supplémentaire.

10. Utilisation des cellules selon l'une quelconque des revendications 4 à 6 ou de la culture de cellules selon l'une quelconque des revendications 7 à 8 servant à développer des systèmes multicellulaires similaires à des tissus ou similaires à des organes in vitro.

11. Utilisation des cellules selon l'une quelconque des revendications 4 à 6 ou de la culture de cellules selon l'une quelconque des revendications 7 à 8 en tant que système in vitro pour tester des produits chimiques, en particulier pour cribler des principes actifs médicamenteux, ou en tant que système in vitro pour la production de substances souhaitées in vitro.

12. Utilisation selon la revendication 11, **caractérisée en ce que** des protéines, en particulier des enzymes présentant une activité optimale à des températures basses, des huiles de poisson ou des acides gras sont produits.

13. Utilisation des cellules selon l'une quelconque des revendications 4 à 6 ou de la culture de cellules selon l'une quelconque des revendications 7 à 8 pour le clonage reproductif de vertébrés à sang froid, en particulier pour l'élevage de poissons, ou en tant que cellules réceptrices ou véhicules servant à recevoir un matériel génétique étranger ou d'autre matériel étranger.

14. Jeu, comprenant les cellules selon l'une quelconque des revendications 4 à 6, ainsi que d'autres agents auxiliaires ou réactifs, par exemple des réactifs servant à la mise en culture des cellules ou des réactifs de diagnostic.
